(19) Europäisches Patentamt
European Patent Office
Office européen des brevets

(11) EP 0 925 781 B1

(12) FASCICULE DE BREVET EUROPEEN

(45) Date de publication et mention de la délivrance du brevet: **09.04.2003 Bulletin 2003/15**

(51) Int Cl.[7]: **A61K 7/48**

(21) Numéro de dépôt: **98403097.3**

(22) Date de dépôt: **09.12.1998**

(54) **Composition dépigmentante**

Hautdepigmentierungsmittel

Skin whitening composition

(84) Etats contractants désignés:
**AT BE CH CY DE DK ES FI GB IE IT LI LU MC NL PT SE**

(30) Priorité: **24.12.1997 FR 9716475**

(43) Date de publication de la demande: **30.06.1999 Bulletin 1999/26**

(73) Titulaire: **CASTER**
**75008 Paris (FR)**

(72) Inventeur: **Rodelet, Jean-François**
**92100 Boulogne-Billancourt (FR)**

(74) Mandataire: **Casalonga, Axel**
**BUREAU D.A. CASALONGA - JOSSE**
**Paul-Heyse-Strasse 33**
**80336 München (DE)**

(56) Documents cités:
**WO-A-96/40047**
**US-A- 5 300 657**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 925 781 B1

Printed by Jouve, 75001 PARIS (FR)

## Description

La présente invention concerne une composition cosmétique ou dermatologique dépigmentante contenant un nouveau principe actif dépigmentant, et son utilisation pour l'éclaircissement de la peau ou le traitement de taches pigmentaires.

La maîtrise des phénomènes pigmentaires cutanés prend une place de plus en plus importante au sein de la recherche cosmétologique. En effet, pour des raisons socioculturelles ou esthétiques, on constate un engouement croissant pour les produits cosmétiques destinés à éclaircir la couleur de la peau ou à en supprimer ou atténuer des taches dues à des désordres pigmentaires.

La pigmentation de la peau et des phanères des mammifères et en particulier de l'homme est due à la présence de mélanines, pigments polymères complexes dont la couleur peut varier du brun-noir (eumélanine) au rouge (phaeomélanine). La biosynthèse de la mélanine, la mélanogenèse, est assurée par des cellules spécialisées appelées mélanocytes qui se trouvent en grand nombre dans les yeux, les bulbes pileux et la peau. Les différentes étapes de la mélanogenèse sont aujourd'hui relativement bien connues.

Dans les différentes étapes de la mélanogenèse, seules les deux premières réactions sont sous le contrôle d'une métalloenzyme, appelée tyrosinase, qui assure l'oxydation de la tyrosine en dihydroxyphénylalanine (DOPA), puis en dopaquinone. Toutes les étapes ultérieures se font spontanément, sans l'intervention d'une enzyme. Par conséquent, si l'on souhaite inhiber ou stimuler la mélanogenèse, la régulation la plus aisée passe par une modification de l'activité de la tyrosinase.

On connaît un certain nombre d'actifs dépigmentants qui empêchent la biosynthèse des mélanines en agissant sur la tyrosinase. Les uns, tels que l'acide kojique, suppriment la synthèse ou la maturation de la tyrosinase par chélation des ions de cuivre indispensables à son fonctionnement. Les autres inhibent directement l'activité de la tyrosinase en se comportant comme des analogues de substrat, occupant le site actif de l'enzyme en compétition avec la tyrosine ou le DOPA. C'est probablement le cas de l'hydroquinone, actif dépigmentant bien connu dont l'utilisation est aujourd'hui strictement réglementée en raison de sa forte cytotoxicité, ainsi que celui de l'arbutine (4-hydroxyphényl-β-D-glucopyranoside), composé dépigmentant extrait d'un certain nombre de plantes (voir brevet français 94 08750).

La demande de brevet japonais n° 63-8317 divulgue un agent à usage externe présentant un pouvoir éclaircissant de la peau et qui contient, comme principe actif, l'acide chélidonique. Cet acide, présent dans différentes plantes et en particulier extractible de la chélidoine, peut être préparé depuis longtemps par synthèse organique à partir d'acétone et d'oxalate d'éthyle (E. R. Riegel et M. C. Reinhard, *Journal of American Chemical Society* (1926), *48*, pages 1334 - 1344, et J. Duclaux et J. Barbière, *Bulletin de la Société Chimique de France* (1933), *53*, pages 564 - 569).

Les présents inventeurs ont constaté de manière inattendue que le principal composé intermédiaire de synthèse, l'acétone-α,α'-dioxalate de diéthyle, présentait des propriétés inhibitrices de la tyrosinase et qu'il convenait comme principe actif dans des compositions dépigmentantes.

L'objet de la présente invention est par conséquent une composition cosmétologique ou dermatologique dépigmentante contenant, comme principe actif, un composé de type acétone-α,α'-dioxalate et son utilisation pour l'éclaircissement de la peau ou le traitement de taches pigmentaires.

Un autre objet de la présente invention consiste en un procédé d'éclaircissement de la peau ou un procédé de traitement cosmétique ou dermatologique de taches pigmentaires, utilisant la composition définie ci-dessus.

D'autres objets de l'invention apparaîtront à la lumière de la description et des exemples qui suivent.

Les compositions cosmétiques ou dermatologiques de la présente invention sont caractérisées en ce qu'elles comprennent, dans un milieu physiologiquement acceptable, un composé de formule générale (I)

(I) 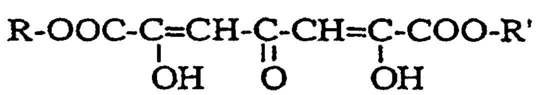

dans laquelle les groupes R et R' représentent, indépendamment l'un de l'autre, un groupe alkyle en $C_{1-6}$ ou un atome d'hydrogène, ou un sel physiologiquement acceptable d'un tel composé.

Dans un mode de réalisation préféré de la présente invention, les deux radicaux R et R', identiques ou différents, représentent un groupement alkyle, de préférence en $C_{1-6}$. On préfère en particulier l'acétone-α,α'-dioxalate de diéthyle, à savoir un composé de formule (I) dans lequel les deux résidus R représentent un groupe éthyle.

Les compositions conformes à la présente invention se présentent de préférence sous forme de lait, de crème ou de gel.

Elles contiennent de préférence le principe actif sous forme vectorisée, c'est-à-dire indu dans des vecteurs tels que des liposomes.

Le principe actif de formule (I) est présent dans les compositions de la présente invention à des concentrations

comprises entre 0,1 et 5 % en poids, de préférence entre 0,25 à 3 % en poids.

Le milieu physiologiquement acceptable des compositions est constitué principalement d'eau. Des additifs habituellement utilisés en cosmétique ou dermopharmacie peuvent y être incorporés. Ces additifs sont par exemple des huiles végétales ou minérales, (des liposomes), des émulsionnants, des épaississants, des conservateurs, des colorants, des parfums, des antiseptiques, des agents régulateurs de pH etc.

L'efficacité du nouveau principe actif de la présente invention a été testée *in vitro* par mesure de son pouvoir inhibiteur de la réaction enzymatique tyrosinase-tyrosine et de la réaction tyrosinase-DOPA, et par évaluation de son pouvoir dépigmentant sur des explants de peau humaines.

L'effet dépigmentant *in vivo* a été déterminé selon la méthode classique de la mesure de la pigmentation de queues de souris noires.

Pour les essais *in vivo* et *in vitro* sur du matériel vivant, on a utilisé comme substance de référence l'hydroquinone, actif dépigmentant connu présentant une certaine cytotoxicité. Dans tous les cas, le principe actif dépigmentant préféré de la présente invention, s'est avéré être d'une efficacité équivalente, voir supérieure, à celle de hydroquinone, et ceci pour des concentrations appliquées inférieures à celles de l'hydroquinone.

L'évaluation de la toxicité du principe actif de la présente invention, administré par voie orale à des rats, donne des valeurs de dose létale à 50 % ($DL_{50}$) supérieures à 2000 mg/kg. L'acétone-$\alpha,\alpha'$-dioxalate de diéthyle n'est donc pas classé comme produit à risque.

Un autre avantage du principe actif préféré de la présente invention, outre sa bonne efficacité et l'absence de toxicité, réside dans la facilité de préparation de celui-ci. Il s'agit en effet d'un protocole de synthèse organique simple dans un récipient unique mettant en oeuvre des composés chimiques peu coûteux tels que l'acétone et l'oxalate d'éthyle, et donnant de bons rendements (70 %). En ceci, il se distingue avantageusement de principes actifs dépigmentants extraits de matériaux végétaux tels que l'arbutine, dont l'extraction nécessite des moyens importants et fournit de faibles quantités de principe actif.

Les exemples suivants sont destinés à illustrer l'invention sans pour autant présenter un caractère limitatif.

**Exemple de préparation**

Préparation de l'acétone-$\alpha,\alpha$-dioxalate de diéthyle

Dans un réacteur de 2 litre, muni d'un système d'agitation et d'un réfrigérant à reflux et chauffé par un bain-marie, on ajoute successivement 58 g d'acétone, 150 g d'oxalate de diéthyle et 273 g d'une solution obtenue par dissolution de 136 g d'éthylate de sodium dans 410 g d'éthanol absolu anhydre (solution A). On chauffe le mélange réactionnel à 75 °C. Après quelques minutes, la solution devient trouble. On ajoute alors 160 g d'oxalate de diéthyle supplémentaire et le reste de la solution A réchauffée, si nécessaire, à 60 °C. On poursuit une agitation vigoureuse du mélange tout en maintenant la température à 75 °C. Il se forme une importante quantité d'un précipité jaune verdâtre qui aboutit à une prise en masse pratiquement totale. On laisse refroidir le mélange réactionnel, puis on y ajoute un mélange de 300 ml d'acide chlorhydrique concentré (d = 1,18) et de 800 g de glace pilée. On agite ce mélange pendant au moins 2 heures afin de neutraliser l'éthanolate de sodium.

On filtre le précipité sur un verre fritté et on lave trois fois avec 1 litre d'eau glacée faiblement acide (pH ≈ 6), puis une fois avec 1 litre d'eau glacée à pH 7.

On laisse sécher le précipité pendant 8 jours à température ambiante.

On obtient ainsi 180 g d'un précipité (70 % de rendement) de couleur jaune-beige.

RMN$^1$H:

déplacement chimique en ppm par rapport au TMS (250 MHz dans du $CDCl_3$): 1,40 (6H, triplet), 4,30 - 4,45 (4 H, quadruplet), 6,38 (2 H, singulet);

RMN$^{13}$C

$\delta$ en ppm par rapport au TMS (300 MHz dans du $CDCl_3$): 14,147; 62,849; 104,100; 161,611; 162,131; 196, 496;

**Exemples de formulation**

**Exemple 1**

| crème dépigmentante I | g pour 100 g |
|---|---|
| acétone-$\alpha,\alpha'$-dioxalate de diéthyle<br>eau déminéralisée | 3,00<br>70,00 |

(suite)

| crème dépigmentante I | g pour 100 g |
| --- | --- |
| huile de sésame | 17,00 |
| cétéaryl-glucoside | 3,00 |
| alcool cétylique | 2,00 |
| lanoline | 1,50 |
| huile de coco hydrogénée | 1,50 |
| oléate de sorbitol-PEG-9 | 1,20 |
| conservateur | 0,50 |
| gomme xanthane | 0,30 |
| parfum | q. s. |

**Exemple 2**

| lait dépigmentant II | g pour 100 g |
| --- | --- |
| liposomes dosés à 5 % d'actif | 20,00 |
| eau déminéralisée | 61,70 |
| huile de vaseline | 15,00 |
| alcool cétylique | 2,00 |
| conservateur | 0,50 |
| parfum | q. s. |

**Exemple 3**

| lait dépigmentant III | g pour 100 g |
| --- | --- |
| liposomes dosés à 5 % d'actif | 5,00 |
| eau déminéralisée | 61,30 |
| huile de vaseline | 25,00 |
| isostéarate de sorbitol et de glycérol-polyoxyéthylène | 2,50 |
| cire d'abeille | 2,50 |
| propylèneglycol | 2,00 |
| sulfate de magnésium | 0,70 |
| oléate de sorbitol-PEG 7 | 0,50 |
| conservateur | 0,50 |
| parfum | q. s. |

**Exemple 3**

| lait dépigmentant IV | g pour 100 g |
| --- | --- |
| liposomes dosés à 5 % d'actif | 10,00 |
| eau déminéralisée | 69,50 |
| huile de vaseline | 10,00 |
| stéarate de glycérol S. E. | 5,50 |
| lanoline | 2,00 |
| vaseline | 2,00 |

(suite)

| lait dépigmentant IV | g pour 100 g |
|---|---|
| conservateur | 0,50 |
| Carbomer | 0,25 |
| triéthanolamine | 0,25 |
| parfum | q. s. |

Pour la préparation de ces quatre produits émulsifiés, on utilise un procédé classique de réalisation d'émulsions. L'eau d'une part et les corps gras d'autre part sont chauffés séparément à 80 °C. On réunit ensuite les deux parties sous agitation dans un agitateur (de type turbine ou mélangeur planétaire) tout en refroidissant. Lorsque la température atteint 30 °C- 40 °C, on ajoute l'actif, soit sous forme pure (émulsion dépigmentante I) soit sous forme vectorisée (émulsions dépigmentantes II - IV), ainsi que le parfum. L'agitation est poursuivie jusqu'à refroidissement complet.

Les produits obtenus sont de couleur jaune pâle à beige clair.

Les liposomes contenant 5 % d'acétone-α,α-dioxalate de diéthyle utilisés pour les exemples de formulation 2 à 4 ont la composition suivante :

| ingrédients | g pour 100 g |
|---|---|
| eau déminéralisée | 56,3 |
| butylèneglycol | 30,5 |
| lécithine de soja | 5,0 |
| acétone-α,α-dioxalate de diéthyle | 5,0 |
| protéine de blé BPM | 2,0 |
| phenonip | 0,5 |
| gomme xanthane | 0,5 |
| microbiocide Coletica | 0,2 |

**Activité inhibitrice *in vitro* de l'acétone-α,α-dioxalate de diéthyle sur la tyrosinase**

I. <u>Inhibition de la réaction L-DOPA - Tyrosinase</u>

L'inhibition de la tyrosinase par l'acétone-α,α'-dioxalate de diéthyle dans la réaction d'oxydation de la L-DOPA en dopaquinone est mise en évidence par chromatographie sur couche mince.

On dépose 10 µl d'une solution d'acétone-α,α'-dioxalate de diéthyle à 1 % dans de l'éthanol (à 96 %) sur des plaques de gel de silice G60. Le solvant de migration est un mélange butanol - eau - acide acétique (4/1/1, v/v/v) que l'on fait migrer jusqu'à une hauteur de 10 cm. Après séchage, on pulvérise sur la totalité de la plaque une solution de L-DOPA (1 M), puis une solution de tyrosine (238 UI/ml). L'exposition à la lumière du jour fait apparaître une bande blanche à un $R_f$ voisin de 0,80 sur fond brun. Cette tache blanche est due à l'absence d'activité tyrosinase convertissant le substrat L-DOPA en dopaquinone, molécule qui se transforme spontanément en présence de lumière en un pigment de couleur marron (fond brun). L'acétone-α,α-dioxalate inhibe donc efficacement la réaction DOPA - tyrosine.

II. <u>Inhibition de la réaction tyrosine - tyrosinase</u>

L'inhibition de la tyrosinase par l'acétone-α,α'-dioxalate de diéthyle dans la réaction d'hydroxylation de la tyrosine en DOPA est mise en évidence par spectrophotométrie d'absorption dans le domaine du visible. Il s'agit d'un dosage spectrophotométrique (absorbance à 477 nm) de la mélanine produite *in vitro* par la tyrosinase à partir du substrat tyrosine.

Afin de montrer que, dans la gamme de concentration étudiée, l'absorbance à 477 nm est directement proportionnelle à l'activité de la tyrosinase, on etablit une courbe d'étalonnage en utilisant des quantités connues croissantes de tyrosinase.

Pour cela, on prépare les solutions d'enzyme et de substrat suivantes :

| - Solution mère de tyrosinase : | |
|---|---|
| tyrosinase (à 2400 UI/mg)<br>tampon bis Tris (0,1 M, pH 6,5)<br>eau - q. s. p. | 10 mg<br>1 ml<br>50 ml |
| - Solution mère de tyrosine : | |
| L-tyrosine<br>eau - q. s. p. | 0,04 g<br>100 ml |

On ajoute à 0 ml, 1,2 ml, 2,0 ml et 2,5 ml de solution de tyrosinase, 2,0 ml de tampon bis Tris (0,1 M, pH 6,5) et 5,0 ml de solution mère de tyrosine et on complète avec de l'eau jusqu'à un volume total de 10,0 ml.

On incube ces mélanges pendant 1 heure et 30 minutes à 37 °C au bain marie, puis on refroidit rapidement à 4 °C. La mesure de l'absorbance des solutions à $\lambda$ = 4,77 nm par rapport à une solution exempte de tyrosinase permet de tracer une droite d'étalonnage caractérisée par l'équation de droite $y = 0{,}813\,x + 1{,}27.10^{-3}$ et par un coefficient de correlation égal à 0,999.

On prépare ensuite les solutions suivantes :

| Solution témoin (100 % d'activité tyrosinase) : | |
|---|---|
| solution mère de tyrosine | 5,0 ml |
| solution mère de tyrosinase (à 96 UI/ml) | 2,0 ml |
| tampon bis Tris (0,1 M, pH 6,5) | 2,0 ml |
| eau - q. s. p | 10,0 ml |

| Solution à doser : | |
|---|---|
| acétone-$\alpha$,$\alpha$'-dioxalate de diéthyle à 2,58 % dans l'alcool | 2,0 ml |
| solution mère de tyrosine | 5,0 ml |
| solution mère de tyrosinase (à 96 UI/ml) | 2,0 ml |
| tampon bis Tris (0,1 M, pH 6,5) | 2,0 ml |

On incube ces deux solutions pendant 1 heure et 30 minutes dans un bain marie à 37 °C. On refroidit rapidement à 4 °C, puis on lit l'absorbance à 477 nm en prenant comme référence un blanc exempt de tyrosinase. Les résultats de cinq essais identiques effectués dans ces conditions sont donnés par le tableau 1 suivant.

Tableau 1

| essai | absorbance à 477 nm solution témoin | absorbance à 477 nm solution à doser |
|---|---|---|
| 1 | 1,23 | ≈ 0 |
| 2 | 1,16 | ≈ 0 |
| 3 | 1,16 | ≈ 0 |
| 4 | 1,23 | ≈ 0 |
| 5 | 1,16 | ≈ 0 |
| moyenne | ≈ 1,16 | ≈ 0 |

Ces résultats montrent que l'acétone-$\alpha$,$\alpha$'-dioxalate de diéthyle inhibe efficacement *in vitro* la production de mélanine par la tyrosinase à partir de la L-tyrosine.

**Pouvoir dépigmentant de l'acétone-$\alpha$,$\alpha$'-dioxalate de diéthyle *in vitro* sur des explants de peau**

L'activité inhibitrice de l'acétone-$\alpha$,$\alpha$'-dioxalate de diéthyle sur la tyrosinase dans les mélanocytes a été évaluée par une étude histologique sur des explants de peau.

Après décongélation et fixation pendant 30 minutes dans une solution de formaldéhyde à 10 % (p/v), on incube des explants de peau humaine provenant d'une plastie abdominale pendant 15 heures à 37 °C en présence

de 0,005 M de L-DOPA et des produits à tester.

L'acétone-α,α'-dioxalate de diéthyle est utilisé à des concentrations de 0,5 %, 1 % et 2 % (p/v) dans une solution de DMSO à 10 % (v/v) dans de l'eau.

On utilise comme produit de référence l'hydroquinone, un inhibiteur connu de la tyrosinase, à une concentration de 1,1 % (p/v) dans une solution de DMSO à 10 % (v/v) dans de l'eau.

Des explants témoins incubés uniquement en présence de 0,005 M de L-DOPA sont réalisés en parallèle.

Pour l'examen histologique, on effectue des coupes d'une épaisseur de 4 µm sur des explants inclus dans de la paraffine et on colore les coupes avec du crésyl violet. '

Les clichés photographiques en microscopie optique en lumière blanche permettent une évaluation visuelle de la coloration des mélanocytes réflétant l'activité de la tyrosinase. Les résultats sont résumés dans le tableau 2 suivant

Tableau 2

| produit | activité de la tyrosinase |
|---|---|
| témoin (absence d'inhibiteur) | +++ |
| hydroquinone à 1,1 % | 0 |
| acétone-dioxalate de diéthyle | |
| à 0.5 % | 0 |
| à 1,0 % | 0 |
| à 2,0 % | 0 |
| +++ : réaction d'oxydation de la DOPA très intense | |
| 0 : absence de réaction d'oxydation de la DOPA | |

Il apparaît que, dans les conditions de l'étude, l'acétone-α,α'-dioxalate de diéthyle testé à raison de 0,5 %, 1,0 % et 2 % inhibe totalement l'activité de la tyrosinase.

**Pouvoir dépigmentant de l'acétone-α,α'-dioxalate de diéthyle *in vivo* sur des queues de souris noires**

L'efficacité de dépigmentation de l'acétone-α,α'-dioxalate de diéthyle a été testée pour deux compositions contenant le principe actif sous forme vectorisée dans des liposomes, en comparaison avec un produit du commerce réputé efficace contenant de l'hydroquinone (Correcteur anti-taches Liérac - réf. L 908/1)

Composition 1 : gel contenant 99,0 % de liposomes dosés à 0,5 % d'acétone-α,α'-dioxalate de diéthyle
Composition 2 : émulsion contenant 10,0 % de liposomes dosés à 0,5 % d'acétone-α,α'-dioxalate de diéthyle
Produit de réf. : composition contenant 2 % d'hydroquinone

Chaque composition est appliquée quotidiennement pendant 28 jours sur la queue de quatre souris noires. Pour chaque souris, on mesure au jour J0, J15 et J28 le paramètre de clarté L* au moyen d'un chromamètre.

L'efficacité (en %) de chaque produit est calculée selon la formule suivante :

$$E\ \% = ((E_{JY} - E_{J0})/E_{J0})\ 100$$

Le tableau 3 ci-dessous présente les valeurs du paramètre L* obtenues pour chaque produit ainsi que l'efficacité (E %) au bout de 15 et 28 jours de traitement. Chaque valeur représente la moyenne ± écart type calculée pour 4 souris.

Tableau 3

| | produit de référence (2 % d'hydroquinone) | | | composition 1 (0,05 % de princ. actif) | | | composition 2 (0,5 % de princ. actif) | | |
|---|---|---|---|---|---|---|---|---|---|
| jour | J0 | J15 | J28 | J0 | J15 | J28 | J0 | J15 | J28 |
| L* moyen | 43,2 | 45,5 | 44,6 | 42,8 | 45,8 | 46,8 | 39,8 | 45,3 | 47,1 |
| ± écart type | ±1,5 | ±1,9 | ±2,1 | ±0,8 | ±0,4 | ±0,8 | ±1,0 | ±0,6 | ±0,8 |
| **E %** | | **5,3** | **3,2** | | **7,1** | **9,5** | | **13,8** | **18,3** |

Ces résultats montrent que les deux compositions renfermant le principe actif de la présente invention donnent

de meilleurs résultats que le produit de la technique antérieure et ceci pour des concentrations d'application inférieures. On constate également que l'acétone-α,α'-dioxalate de diéthyle agit de façon dépendante de la dose, une concentration dix fois plus importante augmentant l'efficacité d'un facteur deux.

Il est bien entendu que la description qui précède n'a été donnée qu'à titre purement illustratif et non limitatif et que des variantes ou des modifications peuvent y être apportées dans le cadre de la présente invention.

## Revendications

1. Composition cosmétique ou dermatologique, **caractérisée en ce qu'**elle comprend, dans un milieu physiologiquement acceptable, un composé de formule générale (I)

(I) 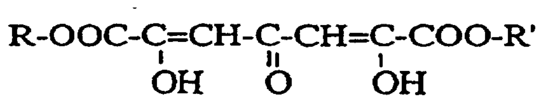

dans laquelle les groupes R et R' réprésentent, indépendamment l'un de l'autre, un groupe alkyle en $C_{1-6}$ ou un atome d'hydrogène, ou un sel physiologiquement acceptable d'un tel composé.

2. Composition selon la revendication 1, **caractérisée en ce que** les deux radicaux R et R' représentent un groupe alkyle.

3. Composition selon la revendication 2, caracérisée en ce que les deux radicaux R et R' représentent un groupe éthyle.

4. Composition selon l'une quelconque des revendications 1 à 3, **caractérisée en ce qu'**elle se présente sous forme de lait, de crème ou de gel.

5. Composition selon l'une quelconque des revendications 1 à 4, **caractérisée en ce qu'**elle contient le composé de formule (I) de préférence sous forme vectorisée dans des liposomes.

6. Composition selon l'une quelconque des revendications 1 à 5, **caractérisée en ce qu'**elle contient le composé de formule (I) à raison de 0,1 à 5 % en poids, de préférence à raison de 0,25 à 3 % en poids.

7. Composition selon l'une quelconque des revendications 1 à 6 **caractérisée en ce que** le milieu physiologiquement acceptable est constitué d'eau et peut comprendre d'autres adjuvants cosmétiques ou dermatologiques tels que des huiles végétales ou minérales, des émulsionnants, des épaississants, des conservateurs, des colorants, des parfums, des antiseptiques, des agents régulateurs de pH ou autres adjuvants similaires.

8. Procédé d'éclaircissement de la peau ou de traitement cosmétique de taches cutanées, **caractérisé par le fait que** l'on applique sur la peau une composition selon l'une quelconque des revendications 1 à 7.

9. Utilisation de la composition définie selon l'une quelconque des revendications 1 à 5 pour la préparation d'une formulation dermatologique destinée au traitement de taches dues à des désordres pigmentaires.

## Claims

1. Cosmetic or dermatological composition,
**characterized in that** it comprises, in a physiologically acceptable medium, a compound of general formula (I)

(I) 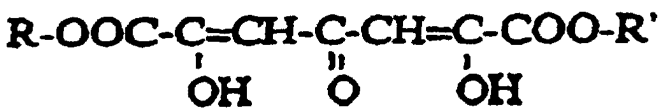

in which the groups R and R' represent, independently of each other, a $C_{1-6}$ alkyl group or a hydrogen atom, or a physiologically acceptable salt of such a compound.

2. Composition according to Claim 1, **characterized in that** the two radicals R and R' represent an alkyl group.

3. Composition according to Claim 2, **characterized in that** the two radicals R and R' represent an ethyl group.

4. Composition according to any one of Claims 1 to 3, **characterized in that** it is in the form of a milk, a cream or a gel.

5. Composition according to any one of Claims 1 to 4, **characterized in that** it contains the compound of formula (I) preferably in vectorized form in liposomes.

6. Composition according to any one of Claims 1 to 5, **characterized in that** it contains the compound of formula (I) in a proportion of from 0.1 to 5% by weight, preferably in a proportion of from 0.25 to 3% by weight.

7. Composition according to any one of Claims 1 to 6, **characterized in that** the physiologically acceptable medium consists of water and can comprise other cosmetic or dermatological adjuvants such as plant or mineral oils, emulsifiers, thickeners, preserving agents, dyes, fragrances, antiseptics, pH regulators or other similar adjuvants.

8. Process for lightning the skin or for the cosmetic treatment of skin marks, **characterized in that** a composition according to any one of Claims 1 to 7 is applied to the skin.

9. Use of the composition defined according to any one of Claims 1 to 5 for the preparation of a dermatological formulation intended for the treatment of marks caused by pigmentation disorders.

**Patentansprüche**

1. Kosmetische oder dermatologische Zusammensetzung, **dadurch gekennzeichnet, dass** diese in einem physiologisch aufnehmbaren Milieu eine Verbindung der allgemeinen Formel (I) enthält:

```
(I)      R-OOC-C=CH-C-CH=C-COO-R'
               |    ||  |
               OH   O   OH
```

wobei die Gruppen R und R' voneinander unabhängig eine Alkylgruppe aus $C_{1-6}$ oder ein Wasserstoffatom, oder ein physiologisch aufnehmbares Salz einer derartigen Verbindung repräsentieren.

2. Zusammensetzung nach Anspruch 1, **dadurch gekennzeichnet, dass** die zwei Radikale R und R' eine Alkylgruppe repräsentieren.

3. Zusammensetzung nach Anspruch 2, **dadurch gekennzeichnet, dass** die zwei Radikale R und R' eine Ethylgruppe repräsentieren.

4. Zusammensetzung nach einem der Ansprüche 1 bis 3, **dadurch gekennzeichnet, dass** sie in Form von Milch, Creme oder Gel vorliegt..

5. Zusammensetzung nach einem der Ansprüche 1 bis 4, **dadurch gekennzeichnet, dass** sie die Verbindung der Formel (I) vorzugsweise vektorisiert in Liposomen enthält.

6. Zusammensetzung nach einem der Ansprüche 1 bis 5, **dadurch gekennzeichnet, dass** sie die Verbindung der Formel (I) in einer Konzentration von 0,1 bis 5 Gewichtsprozent, vorzugsweise in einer Konzentration von 0,25 bis 3 Gewichtsprozent enthält.

7. Zusammensetzung nach einem der Ansprüche 1 bis 6, **dadurch gekennzeichnet, dass** das physiologisch aufnehmbare Milieu im Wesentlichen aus Wasser besteht und sonstige dermatologische Adjuvante aufweisen kann,

beispielsweise pflanzliche oder mineralische Öle, Emulgatoren, Verdickungsmittel, Konservierungsstoffe, Farbstoffe, Duftstoffe, Antiseptika, pH-Wert-Regulatoren oder sonstige ähnliche Adjuvante.

**8.** Verfahren zum Aufhellen der Haut oder zur kosmetischen Behandlung von Hautflecken, **dadurch gekennzeichnet, dass** eine Zusammensetzung nach einem der Ansprüche 1 bis 7 auf die Haut appliziert wird.

**9.** Verwendung der nach einem der Ansprüche 1 bis 5 definierten Zusammensetzung für die Herstellung einer dermatologischen Substanz, die dazu dient, Hautflecken zu behandeln, die auf Pigmentstörungen zurückzuführen sind.